# EUROPEAN PATENT SPECIFICATION

(11) **EP 0 395 287 B1**
(45) Date of publication and mention of the grant of the patent: **29.06.1994**
(21) Application number: 90304103.6
(22) Date of filing: 17.04.1990
(51) Int. Cl.: A61K 7/22

(54) **Dentifrice compositions**
Zahnreinigende Zusammensetzungen
Compositions dentifrices

(30) Priority: 24.04.1989 JP 104151/89
(43) Date of publication of application: 31.10.1990
(73) Proprietor: SUNSTAR KABUSHIKI KAISHA, Takatsuki-shi Osaka-fu (JP)
(72) Inventor: Mori, Shigeki, Takatsuki-shi, Osaka-fu (JP); Makino, Chiho, Takatsuki-shi, Osaka-fu (JP)
(74) Representative: Stoner, Gerard Patrick

(56) References cited:
- GB-A- 2 149 661
- US-A- 3 843 779
- US-A- 3 927 201
- US-A- 3 937 805
- US-A- 4 407 788

## Description

The present invention relates to dentifrice compositions containing bactericide and means for foaming.

Biguanido bactericide and N-alkyldiaminoethylglycine (hereinafter generally referred to as bactericides) are known to be effective for inhibition of the formation of dental plaque, and have hitherto been added to dentifrices. On the other hand, foaming agents (surfactants) and binders added to dentifrices have generally been anionic ingredients. The bactericide tends to react with these ingredients to form a complex, which results in serious loss of bactericidal activity of the bactericide added. Therefore, there is a problem that even when bactericide is added in order to inhibit the formation of dental plaque, in practice its effect is relatively poor.

For solving this problem, compositions employing nonionic surfactant as a foaming agent are disclosed, e.g. in US-A-4,080,441, US-A-4,110,429, US-A-4,118,476 and GB-A-1,573,356. However, there is a further problem that, although such compositions maintain bactericidal activities, their foaming properties and feel in use are adversely affected.

The problem addressed herein is therefore to provide new dentifrice compositions, which desirably combine good activity of the above-mentioned bactericide types with satisfactory foaming properties.

US-A-3937805 discusses a dentifrice having a biguanido bactericide and an N-acyl amino acid surfactant. Use of other non-ionic or amphoteric surfactants is also mentioned.

GB-A-2149661 discusses a dentifrice containing a gelling agent and a polyoxyethylene-polyoxypropylene block copolymer surfactant, and which may contain a biguanido bactericide.

US-A-4407778 similarly discloses a dentifrice containing a gelling agent, and which may contain a biguanido bactericide and a polyoxyethylene-polyoxypropylene block copolymer.

The present inventors have discovered that by using certain new specific surfactant combinations, a dentifrice composition which maintains the stability of bactericidal activities of the bactericide added and has excellent foaming properties may be obtained, and need not necessarily use any binder.

Thus, according to the present invention, there is provided a dentifrice composition comprising
(a) 0.001 to 0.5% by weight of biguanido bactericide and/or N-alkyl-diaminoethylglycine bactericide;
(b) 11% to 80% by weight of polyoxyethylene-polyoxypropylene block copolymer surfactant, and
(c) N-higher acyl amino acid or salt thereof.

The present invention also provides a method of making a dentifrice composition, comprising mixing together
(a) biguanido bactericide and/or N-alkyl-diaminoethylglycine bactericide;
(b) polyoxyethylene-polyoxypropylene block copolymer surfactant, and
(c) N-higher acyl amino acid or salt thereof;
   wherein said composition comprises 0.001 to 0.5% by weight of the bactericide and from 11% to 80% by weight of the polyoxyethylene-polyoxypropylene block copolymer surfactant.

In another aspect, the present invention provides use, in a dentifrice composition comprising 0.001 to 0.5% by weight of biguanido and/or N-alkyldiaminoethylglycine bactericide, of from 11% to 80% by weight of polyoxyethylene-polyoxypropylene block copolymer surfactant and N-higher acyl amino acid, or salt thereof, in combination as foaming agents, to inhibit loss of activity of the bactericide and to gel the composition.

Biguanido bactericides which may be used in the invention include e.g. chlorhexidine hydrochloride, chlorhexidine gluconate, chlorhexidine acetate, and 1,6-bis-(2-ethylhexyl biguanidohexane)dihydrochloride. The N-alkyldiaminoethylglycine may be an N-(C₁₂ to C₁₈)-alkyldiaminoethylglycine. Examples thereof are N-lauryldiaminoethylglycine, N-myristyldiaminoethylglycine, and N-(C₁₂ to C₁₄)alkyldiaminoethylglycine. These bactericides can be used alone or in combination. Salts e.g. hydrochlorides may be suitable. Although the amount of bactericide to be added is not specifically limited, usually it is 0.001 to 0.5% by weight based on the total weight of the dentifrice composition in view of bactericidal activities and economy.

Suitable polyoxyethylene-polyoxypropylene block copolymer surfactants for use herein include e.g. polyoxyethylene-polyoxypropylene glycol represented by the general formula: 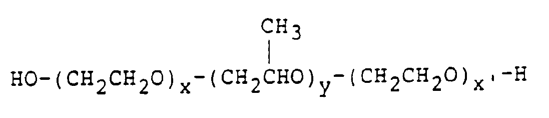
wherein x, x′ and y are integers, and
polyoxyethylene-polyoxypropylene glycol addition compound of ethylenediamine represented by the general formula: 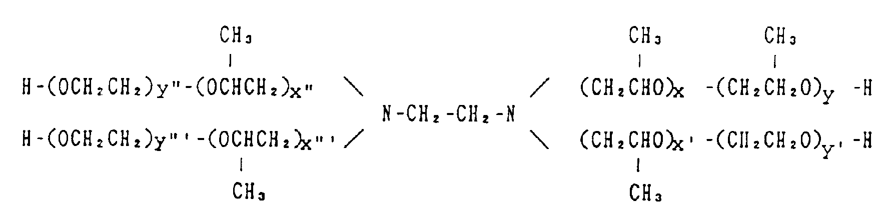
wherein x, x′, x˝, x‴, y, y′, y˝ and y‴ are integers.

Such a block copolymer is commercially available under the trade name of "PLURONIC®"or "TETRONIC®", manufactured by BASF Corporation, U.S.A. In general, the copolymer is chemically defined according to the molecular weight of its hydrophobic portion composed of polyoxypropylene and the proportion (% by weight) of its hydrophilic portion composed of polyoxyethylene in the molecule. Preferred polyoxyethylene-polyoxypropylene glycol has a molecular weight of the hydrophobic group (polyoxypropylene) of 1,400 to 4,000 and contains 30 to 80% by weight of the hydrophilic group (polyoxyethylene) in the molecule. A preferred polyoxyethylene-polyoxypropylene glycol addition compound of ethylenediamine has a molecular weight of the hydrophobic group (polyoxypropylene) of 4,500 to 7,000 and contains 40 to 90% by weight of the hydrophilic group (polyoxyethylene) in the molecule. The polyoxyethylene-polyoxypropylene block copolymer surfactant may be added to the composition in an amount of about 11 to 80% by weight, preferably 15 to 40% by weight, based on the total weight of the dentifrice composition. When the amount of the surfactant added is less than 11% by weight, a problem in form stability due to syneresis tends to arise through insufficient gelation. On the other hand, when the amount exceeds 80% by weight, usually gelation is too strong to obtain a suitable viscosity as a dentifrice.
Examples of the N-higher acylamino acid or salt thereof to be used in the present invention are N-(C₁₂ to C₁₈)-acylamino acids and alkali metal salts thereof. Examples thereof include sodium N-lauroyl glutamate, sodium N-lauroyl sarcosinate, sodium N-myristoyl sarcosinate, N-oleoyl sarcosinate, sodium lauroyl methylalanine. Such a compound can be used alone or in combination thereof. The amount of the compound is usually 0.01 to 5% by weight, preferably 0 1 to 3% by weight, based on the total weight of the dentifrice composition. When the amount is less than 0.01% by weight, the improvement of foaming properties is usually inadequate and, when the amount exceeds 5% by weight, a problem in safety for oral mucosa such as detachment of oral mucosa and the like, is likely.

A dentifrice composition embodying the invention may be prepared in the form of toothpaste, pasta and the like according to the conventional production process. Further, in so far as unacceptable adverse effects are not created, appropriate ingredients, for example, polishing agents such as calcium secondary phosphate anhydride or dihydrate, calcium carbonate, calcium pyrophosphate, insoluble sodium metaphosphate, aluminum hydroxide, aluminum oxide, a resin and the like; humectants such as polyethylene glycol, sorbitol, glycerin, propylene glycol and the like; essential oils such as peppermint, spearmint and the like; flavors such as 1-menthol, carvone, anethole and the like; sweeteners such as saccharin sodium, stevioside, neohesperidyl hydrocharcone, glycyrrhizin, perillartin, p-methoxycinnamic aldehyde, thaumatin and the like; pharmacologically active ingredients such as sodium monofluorophosphate, sodium fluoride, dextranase, mutanase, hinokitiol, allantoin, ε-aminocaproic acid, tranexamic acid, azulene, vitamin E derivatives, sodium chloride and the like may be added as desired.

As described below, dentifrice compositions embodying the invention maintain the stability of bactericidal activities. Therefore, they can effectively inhibit the formation of dental plaque and prevent gingivitis.

The following Examples and Comparative Examples further illustrate the present invention in detail.

In the Examples and Comparative Examples, all the percents are by weight unless otherwise stated.

### Examples 1 to 12 and Comparative Examples 1 to 28

Using the formulations as shown in Tables 1 to 4, toothpastes were prepared according to the conventional method.

Bactericidal activities and foaming properties of the toothpastes thus obtained were determined by the following methods.

### [Bactericidal activity test]

Each toothpaste (about 6.0 g) was weighed and suspended in distilled water. The suspension was centrifuged to obtain the supernatant. The supernatant was diluted with distilled water so that the concentration of bactericide in the supernatant became 0.05%, 0.10% and 0.20% in the case of chlorhexidine gluconate, or 0.001 %, 0.002 % and 0.004 % in the case of N-(C₁₂to C₁₄)alkyldiaminoethylglycine hydrochloride (TEGO 51 manufactured by Goldschmidt Corp.).

Also, chlorhexidine gluconate and diaminoethyl glycine hydrochloride were separately dissolved in distilled water so that the concentration thereof became 0.05%, 0.10% and 0.20% in the case of the former, or 0.001%, 0.002% and 0.004% in the case of the latter. These solutions were used as standard solutions for the determination of minimum bactericidal concentration (%, hereinafter abbreviated as MBC).

To each sample (10 ml) was added 10⁸ to 10⁹ CFU/ml of a Streptococcus mutans cell suspension (0.1 ml) and a bactericidal reaction was carried out in a water bath at 37°C for 15 minutes. After completion of the reaction, one loopful of each sample was spread on a trypticase soy agar (TSA) plate containing 0.5% of polyoxyethylene monooleate and 0.07% of lecithin. Then, it was incubated at 37°C for 2 days under anaerobic conditions (N₂/H₂/CO₂ = 85/10/5) and MBC was determined.

MBC of the standard solutions were 0.05% for chlorhexidine gluconate and 0.001% for N-(C₁₂ to C₁₄)alkyldiaminoethylglycine hydrochloride (TEGO 51).

The samples were evaluated according to the following criteria.
A: MBC of the sample tested was the same as that of the standard solution [chlorhexidine glutamate: 0.05 %, N-(C₁₂ to C₁₄)alkylaminoethylglycine hydrochloride (TEGO 51): 0.01 %].
B: MBC of the sample tested was greater than that of the standard solution.

### [Foaming test]

The toothpaste to be tested was diluted three times with distilled water and the diluted solution (100 ml) was placed in a measuring cylinder (inner diameter: 6.5 mm) and stirred in a water bath at 30°C for 3 minutes by using a contrarotation stirrer (1000 r.p.m., contrarotating every 6 seconds, four bladed screw, 5.0 mm in diameter). The foaming volume was expressed by the difference between the apparent volume just after stirring and the volume of diluted solution before stirring as follows.$\text{Foaming volume (ml) = Apparent volume just after stirring (ml) - Volume of diluted solution before stirring (ml)}$

### [Overall evaluation]

Overall evaluation was conducted according to the following criteria.
A: Evaluation of bactericidal activities was A and the foaming volume was not less than 180 ml.
B: At least, evaluation of bactericidal activities was B or the foaming volume was less than 180 ml.

The results are shown in Tables 1 to 4. In the following Tables. 1 to 4, all the amount of ingredients added are % by weight.

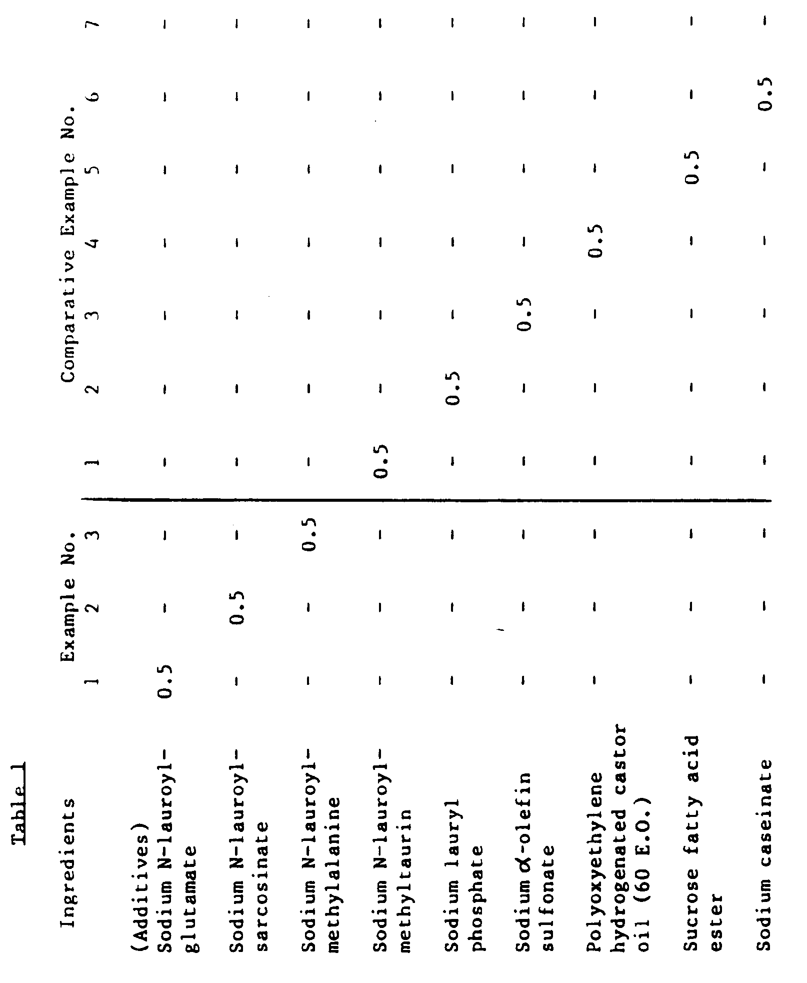

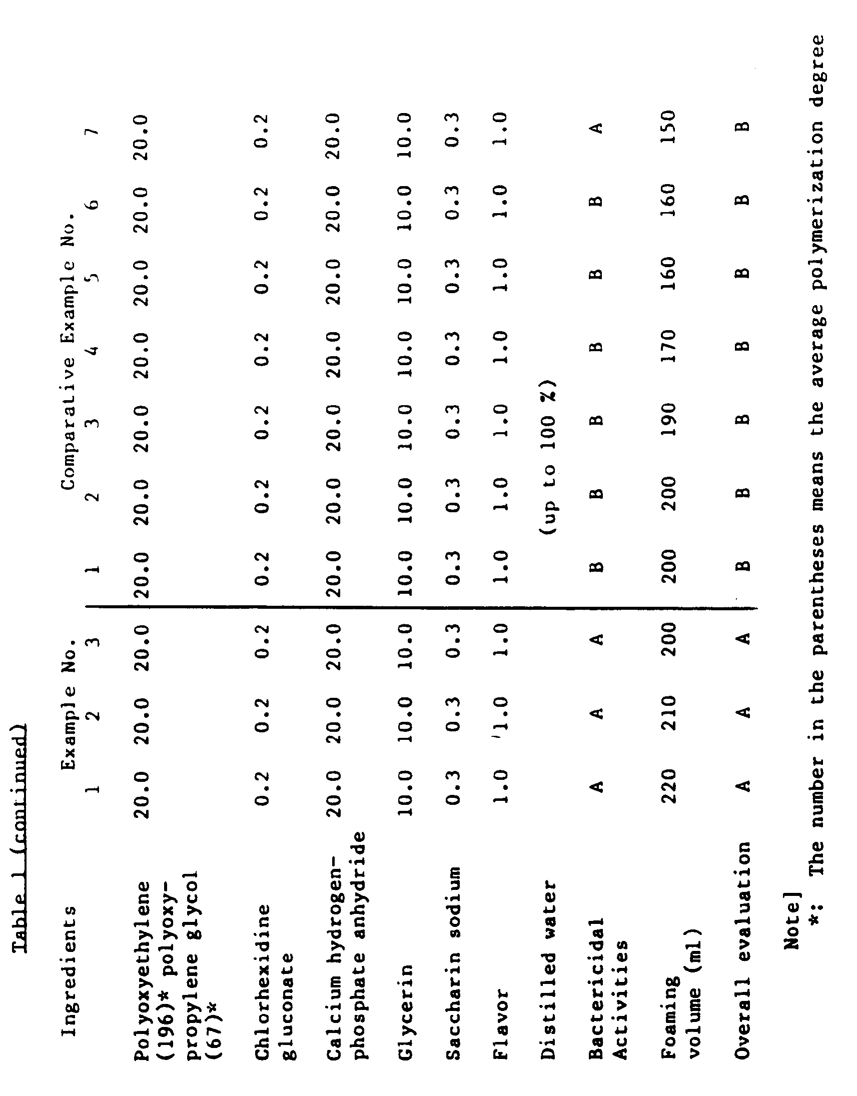

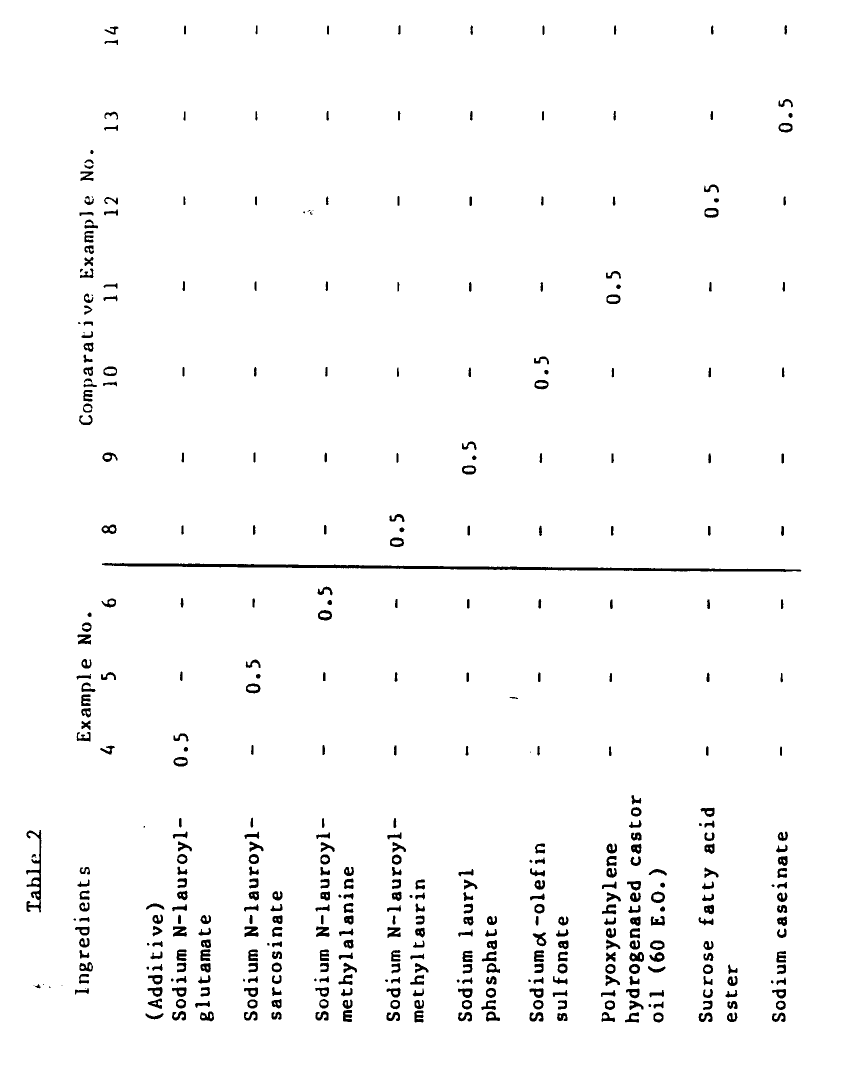

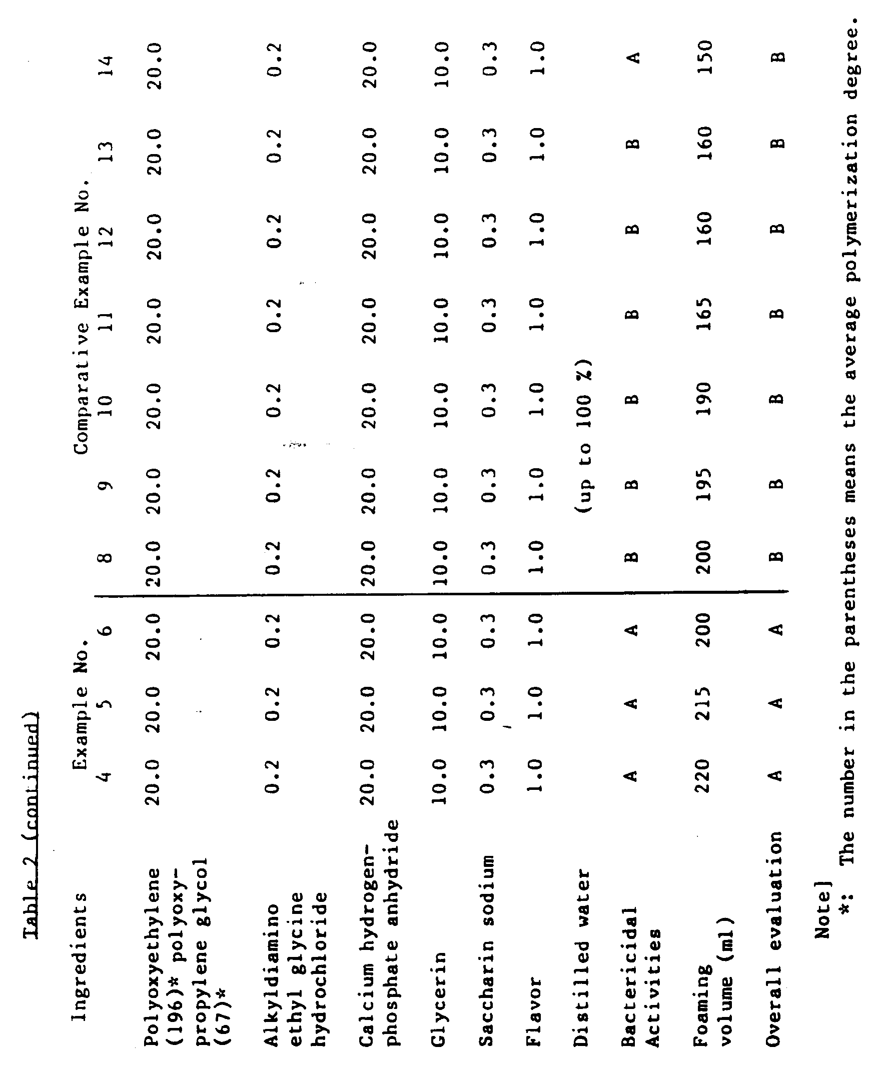

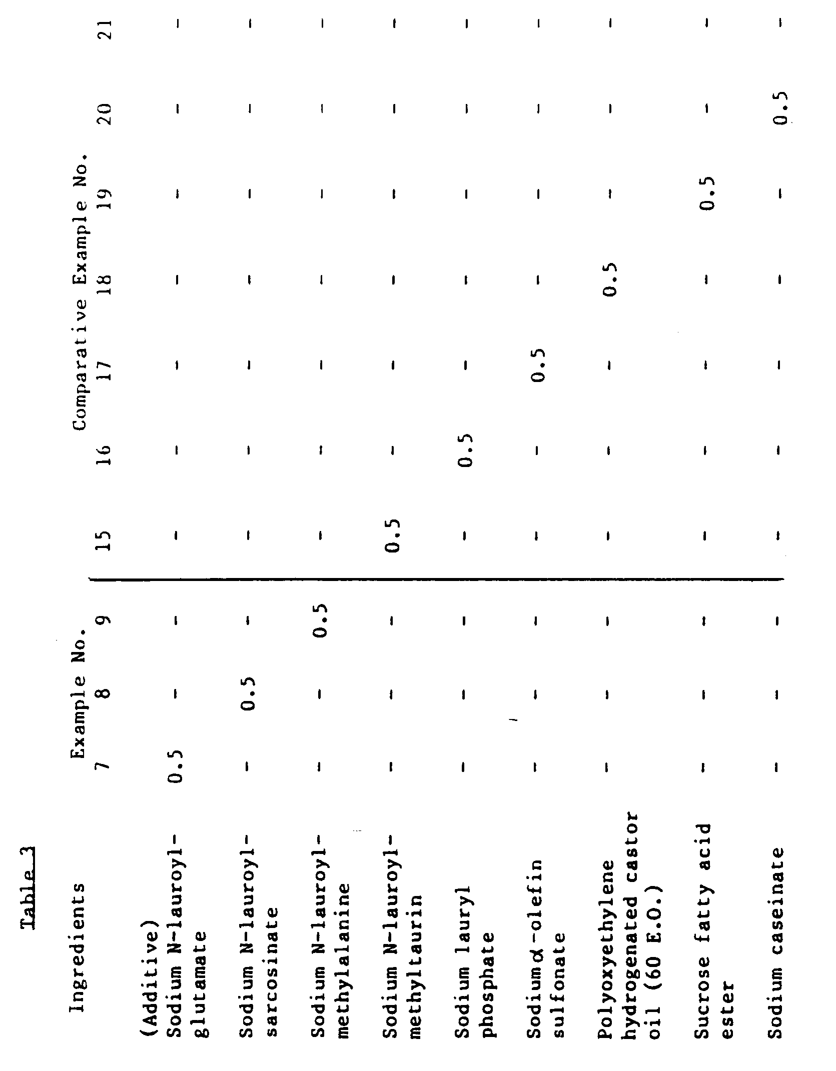

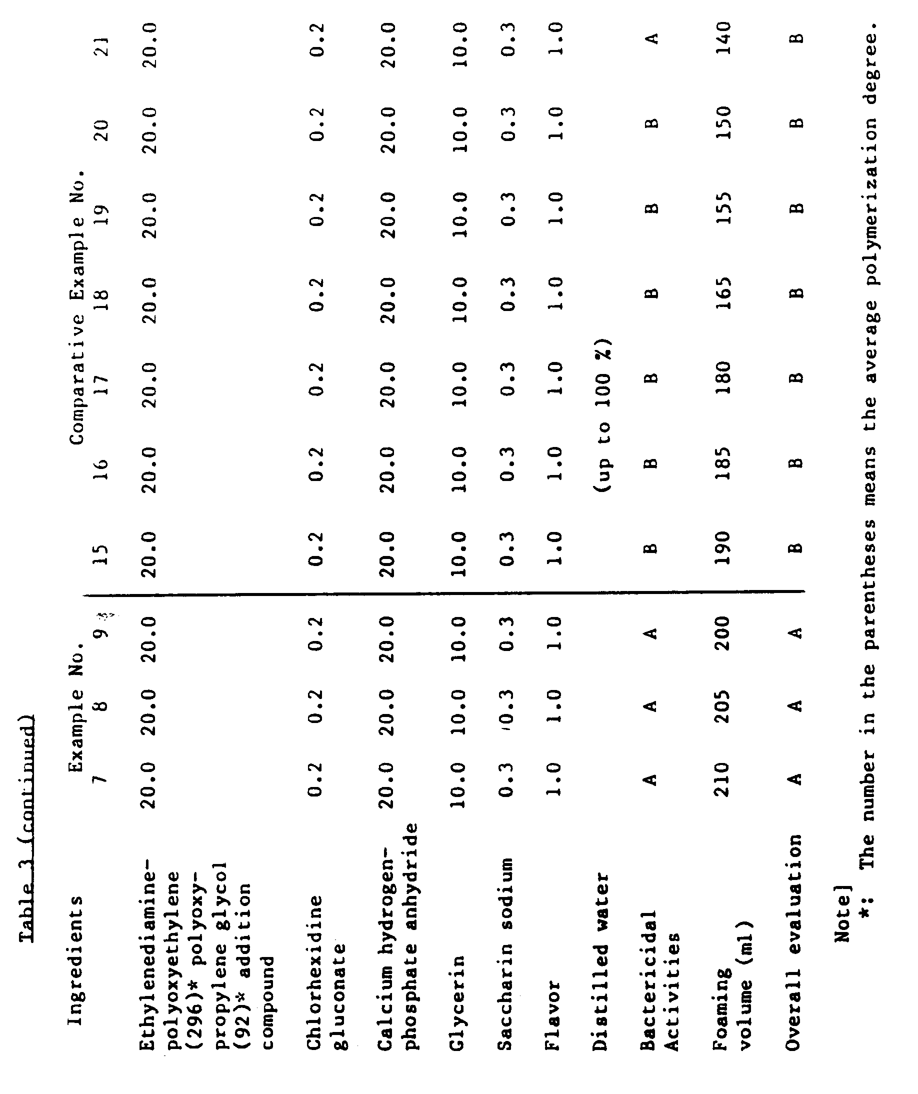

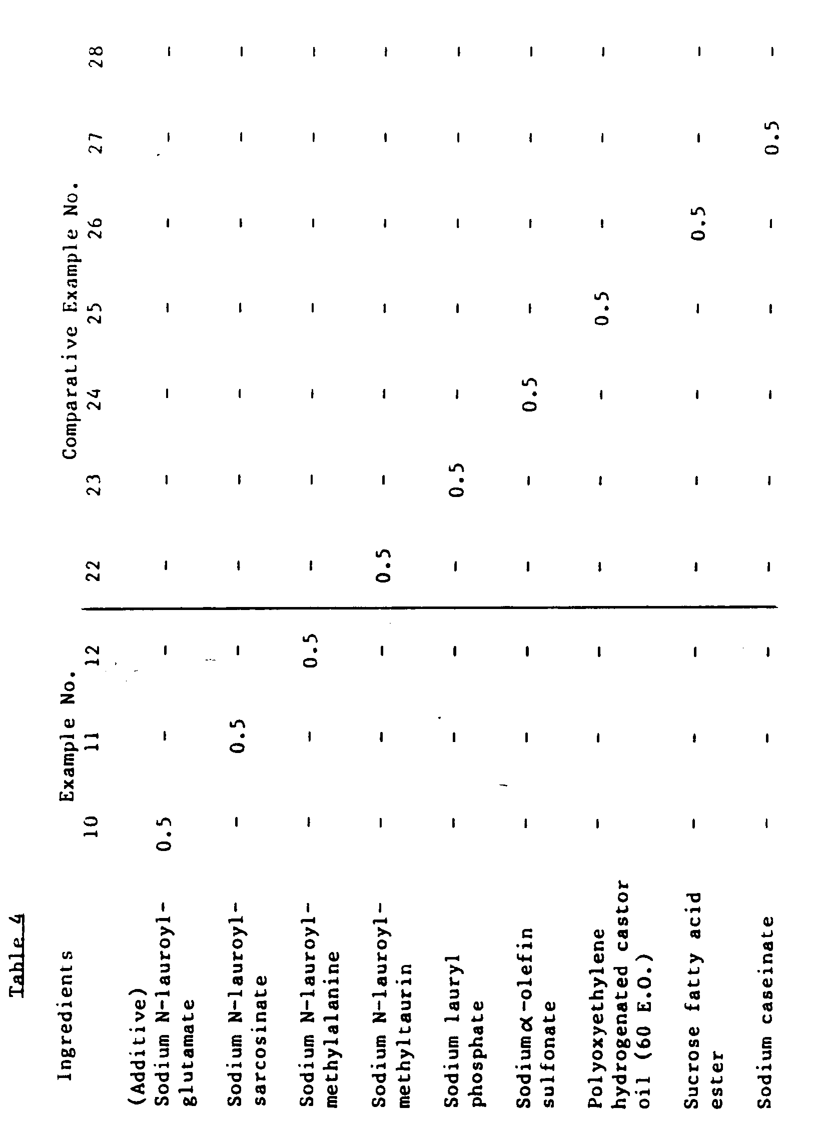

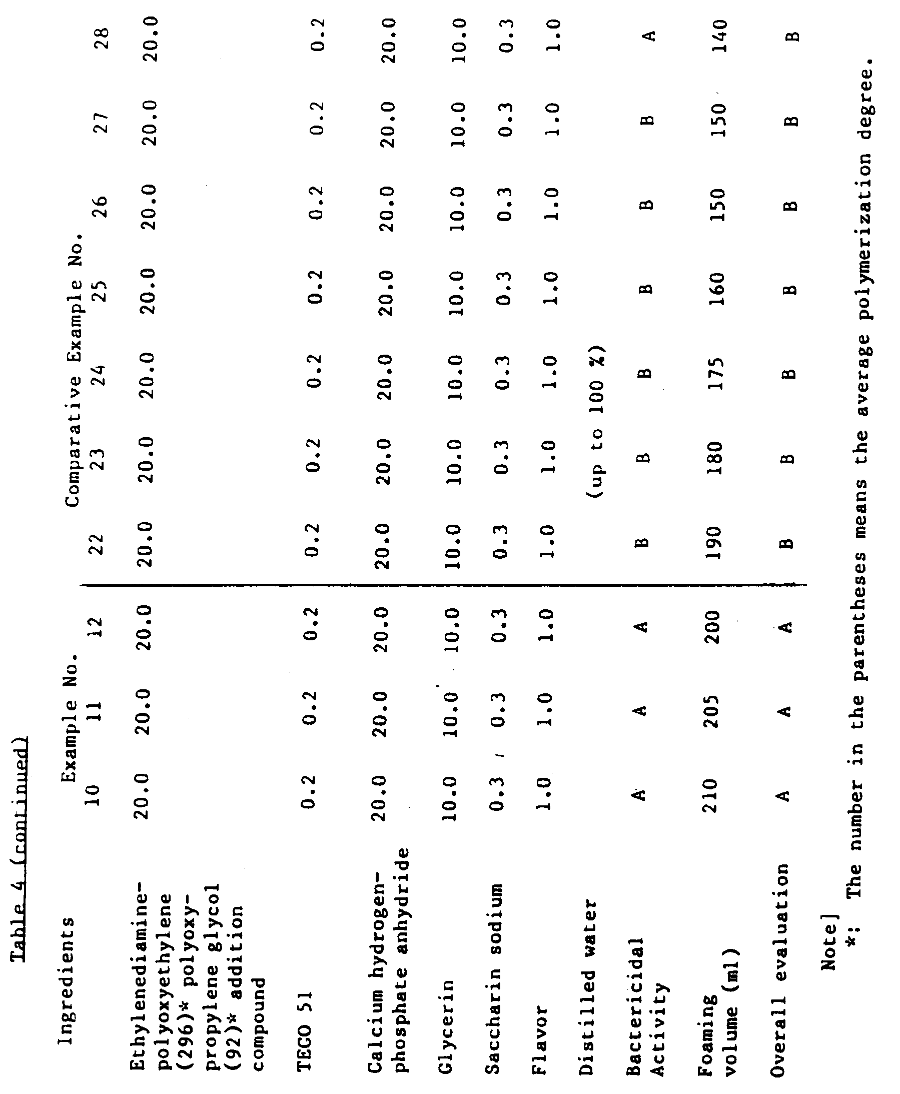

As shown in Tables 1 to 4, by using biguanido bactericides or N-alkyldiaminoethylglycine in combination with the polyoxyethylene polyoxypropylene block copolymer surfactant and N-higher acylamino acid or salt thereof, good foaming properties were obtained without loss of bactericidal activity of the bactericide used.

### Example 13

Using the following formulation, a toothpaste was prepared according to the conventional method.

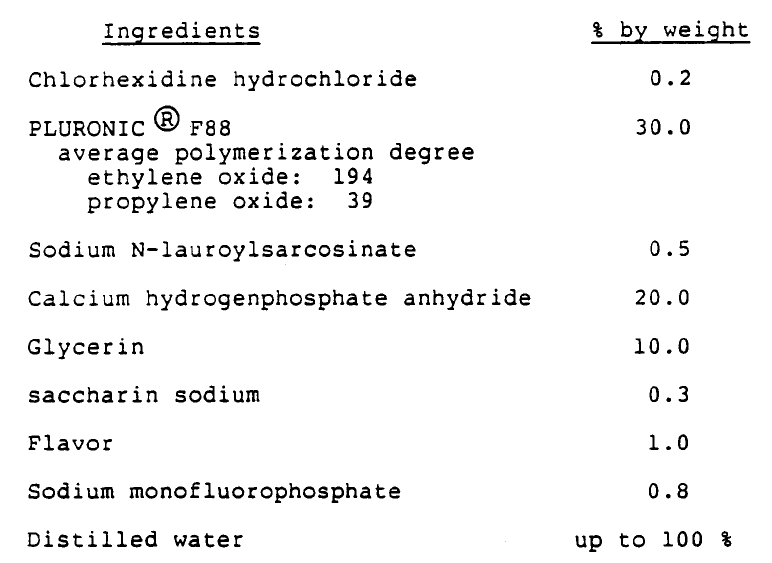

### Example 14

Using the following formulation, a toothpaste was prepared according to the conventional method.

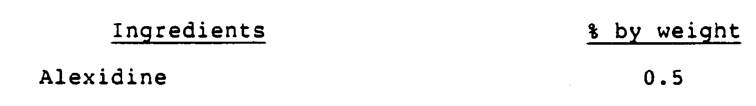

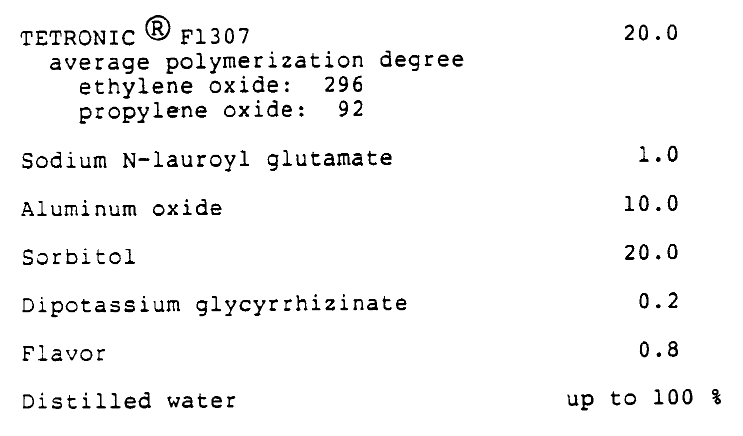

### Example 15

Using the following formulation, a toothpaste was prepared according to the conventional method.

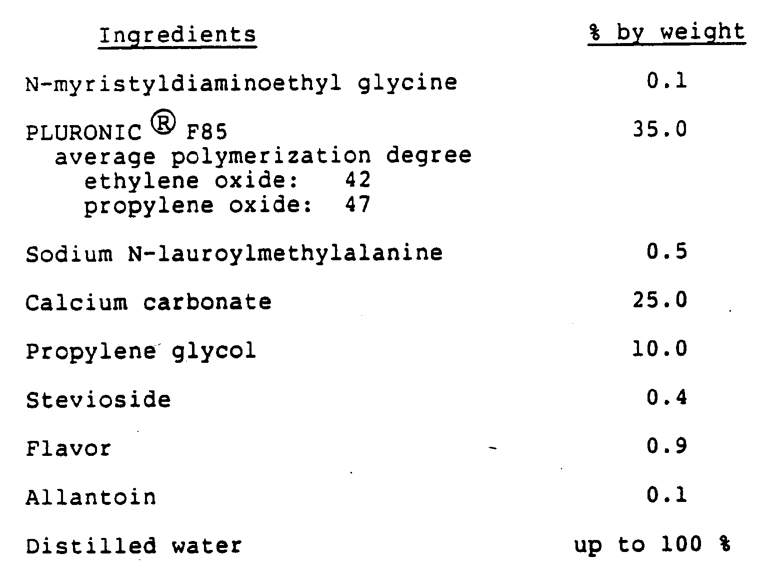

### Example 16

Using the following formulation, a toothpaste was prepared according to the conventional method.

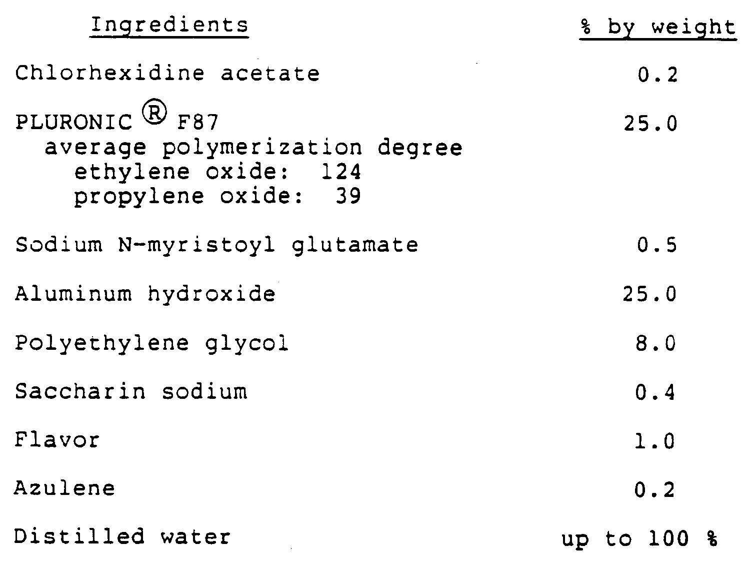

All the compositions of Examples 13 to 16 had good bactericidal activities and foaming properties.

## Claims

1. A dentifrice composition comprising
(a) 0.001 to 0.5% by weight of biguanido bactericide and/or N-alkyl-diaminoethylglycine bactericide;
(b) 11% to 80% by weight of polyoxyethylene-polyoxypropylene block copolymer surfactant, and
(c) N-higher acyl amino acid or salt thereof.

2. A dentifrice composition according to claim 1, wherein the biguanido bactericide is chlorhexidine hydrochloride, chlorhexidine gluconate, chlorhexidine acetate, 1,6-bis-(2-ethylhexyl biguanidohexane) dihydrochloride, or a combination of any of these.

3. A dentifrice composition according to claim 1 or claim 2 wherein the N-alkyldiaminoethylglycine is N-(C₁₂ to C₁₈)-dialkylaminoethylglycine.

4. A dentifrice composition according to any one of the preceding claims wherein the N-higher acylamino acid or salt thereof is N-(C₁₂ to C₁₈)-acylamino acid or an alkali metal salt thereof.

5. A dentifrice composition according to any one of the preceding claims, containing 0.01 to 5% by weight of the N-higher acylamino acid or salt thereof based on the total weight of the composition.

6. A method of making a dentifrice composition, comprising mixing together
(a) biguanido bactericide and/or N-alkyl-diaminoethylglycine bactericide;
(b) polyoxyethylene-polyoxypropylene block copolymer surfactant, and
(c) N-higher acyl amino acid or salt thereof;
wherein said composition comprises 0.001 to 0.5% by weight of the bactericide and from 11% to 80% by weight of the polyoxyethylene-polyoxypropylene block copolymer surfactant.

7. Use, in a dentifrice composition comprising 0.001 to 0.5% by weight of biguanido and/or N-alkyldiaminoethylglycine bactericide, of from 11% to 80% by weight of polyoxyethylene-polyoxypropylene block copolymer surfactant and N-higher acyl amino acid, or salt thereof, in combination as foaming agents, to inhibit loss of activity of the bactericide and to gel the composition.

## Patentansprüche

1. Zahnputzmittel-Zusammensetzung, dadurch **gekennzeichnet**, daß sie
(a) 0,001 bis 0,5 Gew.-% Biguanidobakterizid und/oder N-Alkyldiaminoethylglycinbakterizid;
(b) 11 bis 80 Gew.-% Poloxyethylenpolyoxypropylen-Blockcopolymer als grenzflächenaktives Mittel, und
(c) eine N-höhere Acylaminosäure oder ein Salz davon enthält.

2. Zahnputzmittel-Zusammensetzung nach Anspruch 1, dadurch **gekennzeichnet**, daß sie als Biguanidobakterizid Chlorhexidinhydrochlorid, Chlorhexidingluconat, Chlorhexidinacetat, 1,6-Bis-(2-ethylhexylbiguanidohexan)dihydrochlorid oder ein Gemisch aus irgendwelchen dieser Verbindungen enthält.

3. Zahnputzmittel-Zusammensetzung nach Anspruch 1 oder 2, dadurch **gekennzeichnet**, daß sie als N-Alkyldiaminoethylglycin N-(C₁₂-C₁₈)dialkylaminoethylglycin enthält.

4. Zahnputzmittel-Zusammentzung nach einem der vorhergehenden Ansprüche, dadurch **gekennzeichnet**, daß sie als N-höhere Acylaminosäure oder Salz davon N-(C₁₂-C₁₈)acylaminosäure oder ein Alkalimetallsalz davon enthält.

5. Zahnputzmittel-Zusammensetzung nach einem der vorhergehenden Ansprüche, dadurch **gekennzeichnet**, daß sie 0,01 bis 5 Gew.-% der N-höheren Acylaminosäure oder eines Salzes davon, bezogen auf das Gesamtgewicht der Zusammensetzung, enthält.

6. Verfahren zur Herstellung einer Zahnputzmittel-Zusammensetzung, dadurch **gekennzeichnet**, daß miteinander
(a) ein Biguanidobakterizid und/oder ein N-alkyldiaminoethylglycinbakterizid;
(b) ein Polyoxyethylenpolyoxypropylen-Blockcopolymer als grenzflächenaktives Mittel, und
(c) eine N-höhere Acylaminosäure oder ein Salz davon
vermischt werden, so daß die Zusammensetzung 0,001 bis 0,5 Gew.-% Bakterizid und von 11 bis 80 Gew.-% Polyoxyethylenpolyoxypropylen-Blockcopolymer als grenzflächenaktives Mittel enthält.

7. Verwendung einer Zahnputzmittel-Zusammensetzung, welche 0,001 bis 0,5 Gew.-% eines Biguanido- und/oder N-Alkyldiaminoethylglycinbakterizids, von 11 bis 80 Gew.-% Polyoxyethylenpolyoxypropylen-Blockcopolymer als grenzflächenaktives Mittel und eine N-höhere Acylaminosäure, oder ein Salz davon, im Gemisch als Schäumungsmittel zur Inhibierung des Aktivitätsverlustes des Bakterizids und für die Gelierung der Zusammensetzung enthält.

## Revendications

1. Composition dentifrice comprenant
(a) 0,001 à 0,5 % en masse de bactéricide de type biguanide et/ou de bactéricide N-alkyldiaminoéthylglycine;
(b) 11 à 80 % en masse de tensioactif copolymère séquencé polyoxyéthylène-polyoxypropylène, et
(c) un N-(acyl supérieur)aminoacide ou un de ses sels.

2. Composition dentifrice selon la revendication 1, dans laquelle le bactéricide de type biguanide est le chlorhydrate de chlorhexidine, le gluconate de chlorhexidine, l'acétate de chlorhexidine, le dichlorhydrate de 1,6-(2-éthylhexylbiguanidohexane) ou une de leurs associations.

3. Composition dentifrice selon la revendication 1 ou la revendication 2, dans laquelle la N-alkyldiaminoéthylglycine est la N-(alkyl en C₁₂-C₁₈)diaminoéthylglycine.

4. Composition dentifrice selon l'une quelconque des revendications précédentes, dans laquelle le N-(acyl supérieur)aminoacide ou un de ses sels est le N-(acyl en C₁₂-C₁₈)aminoacide ou un de ses sels de métal alcalin.

5. Composition dentifrice selon l'une quelconque des revendications précédentes, contenant 0,01 à 5 % en masse du N-(acyl supérieur)aminoacide ou d'un de ses sels, par rapport à la masse totale de la composition.

6. Procédé de préparation d'une composition dentifrice comprenant le mélange
(a) du bactéricide de type biguanide et/ou bactéricide N-alkyldiaminoéthylglycine ;
(b) du tensioactif copolymère séquencé polyoxyéthylène-polyoxypropylène, et
(c) du N-(acyl supérieur)aminoacide ou d'un de ses sels;
dans lequel ladite composition contient 0,001 à 0,5 % en masse du bactéricide et de 11 % à 80 % en masse du tensioactif copolymère séquencé polyoxyéthylène-polyoxypropylène.

7. Utilisation, dans une composition dentifrice contenant 0,001 à 0,5 % en masse de bactéricide de type biguanide et/ou N-alkyldiaminoéthylglycine, de 11 % à 80 % en masse du tensioactif copolymère séquencé polyoxyéthylène-polyoxypropylène et du N-(acyl supérieur)aminoacide ou d'un de ses sels, en association en tant qu'agents moussants pour inhiber la perte d'activité du bactéricide et pour gélifier la composition.
